(19) Europäisches Patentamt
European Patent Office
Office européen des brevets

(11) Publication number: **0 504 938 A2**

(12) # EUROPEAN PATENT APPLICATION

(21) Application number: **92104920.1**

(51) Int. Cl.[5]: **A61K 37/02**, C07K 5/08

(22) Date of filing: **20.03.92**

(30) Priority: **22.03.91 JP 59182/91**
**22.03.91 JP 59185/91**

(43) Date of publication of application:
**23.09.92 Bulletin 92/39**

(84) Designated Contracting States:
**AT BE CH DE DK ES FR GB GR IT LI LU MC NL PT SE**

(71) Applicant: **SUNTORY LIMITED**
**1-40, Dojimahama 2-chome**
**Kita-ku, Osaka-shi, Osaka 530(JP)**

(72) Inventor: **Kiso, Yoshinobu**
**12-18, 105, Shimochujou-cho**
**Ibaraki-shi, Osaka(JP)**
Inventor: **Hayashi, Yasuhiro**
**2-2-23, Kujou, Nishi-ku**
**Osaka(JP)**
Inventor: **Higuchi, Naoki**
**2-13-23, 1-305, Ishibashi**
**Ikeda-shi, Osaka(JP)**
Inventor: **Saitoh, Masayuki**
**16-16, 12, Futaba-cho**
**Ibaraki-shi, Osaka(JP)**
Inventor: **Hashimoto, Masaki**
**3-23-9, Shindou**
**Ibaraki-shi, Osaka(JP)**

(74) Representative: **Vossius & Partner**
**Siebertstrasse 4 P.O. Box 86 07 67**
**W-8000 München 86(DE)**

(54) **Prophylactic and therapeutic agent for bone diseases comprising di- or tripeptide derivative as active ingredient.**

(57) Use of a compound represented by the following general formula (I):

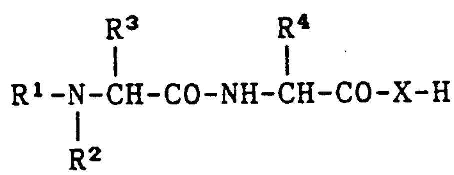

(I)

wherein $R^1$ represents a straight or branched acyl group having 2 to 10 carbon atoms, a branched cyclic or bridged cyclic alkyloxycarbonyl group having 4 to 15 carbon atoms, a substituted or unsubstituted benzyloxycarbonyl group, a 2,2,2-trichloroethyloxycarbonyl group, a 2-(trimethylsilyl)-ethyloxycarbonyl group, a p-toluenesulfonyl group, an o-nitrophenylsulfenyl group, a diphenylphosphonothioyl group, a triphenylmethyl group, a 2-benzoyl-1-methylvinyl group or a 4-phenylbutanoyl group;
$R^2$ represents a hydrogen atom or forms a phthaloyl group together with $R^1$;
$R^3$ represents an isobutyl group, an n-butyl group or an isopropyl group;
$R^4$ represents an n-butyl group or an isobutyl group;
X represents a direct bond, a methionine residue, a leucine residue or a norleucine residue; and
H at the C-terminal means that the carboxyl group is reduced in the form of the aldehyde;
as an active ingredient for the preparation of a pharmaceutical composition useful in preventing or treating malignant hypercalcemia, bone Paget's disease or osteoporosis.

EP 0 504 938 A2

This invention relates to a prophylactic or therapeutic composition for bone diseases such as malignant hypercalcemia, bone Paget's disease and osteoporosis. The present invention has been completed based on a finding that a class of specific di- or tripeptide derivatives having a protease inhibition activity is particularly effective in the prevention and treatment of bone diseases.

In recent years, the rapid increase in the number of aged people in the population has caused an increased in so-called geriatric diseases. Among these diseases, bone diseases including osteoporosis are accompanied by a higher incidence of bone fracture, and this has been the major cause of the increase of aged patients who are bed-ridden. Therefore, there is an urgent need for a pharmaceutical composition for preventing and treating the above-mentioned bone diseases.

Bone is not a tissue which does not undergo any change once formed. It is continuously formed and metabolized. Thus the structure and the amount of bone is maintained due to the balance between osteogenesis and bone resorption. When this balance is lost due to aging or some other factors, various bone diseases are induced. Examples of diseases due to abnormal sthenia of bone resorption include malignant hypercalcemia caused by myeloma or lymphoma, bone Paget's disease caused by local bone resorption and osteoporosis in aged people accompanied by decrease in bone weight, though the etiological causes of this disease are as yet unknown.

Bones mainly comprise collagen fibers (i.e., organic matter ) and calcium salts (i.e., inorganic matter ). These substances are combined and form bones, constructions which are highly resistant against tension and pressure. In particular, calcium salts amount to 70% of the total bone weight. In bone diseases, calcium salts tend to dissolve into the blood and thus are slowly lost, as the diseases proceed. Known methods for preventing or treating these diseases comprise supplying calcium or maintaining the normal calcium level, and in this context active vitamin $D_3$ preparations and calcium preparations have been employed. Further, hormone preparations such as estrogen and calcitonin preparations have been used in order to suppress decalcification of bones.

In addition to the above-mentioned therapeutic methods for preventing decrease in calcium salts, the importance of prevention of the decrease in collagen fibers in bone diseases has been recently noted. Namely, recent studies have revealed that collagen type I, which is the collagen contained in bones as the matrix, is not decomposed by the usual collagenases but is decomposed by cathepsin L which is a thiol protease found among lysosomal enzymes [Nobuhiko Katsunuma, Abstracts of 11th Medicinal Chemistry Symposium, p.128 (1990)]. Further, it has been reported that cathepsin L decomposes collagens type II, IX and XI, which constitute arthrodial cartilages, in a neutral pH range [FEBS Letters, 269 (1), 189 - 193 (1990)-]. It has furthermore been reported that a cysteine protease inhibitor suppresses bone resorption [Biochemical Journal, 192, 365 - 368 (1980); Biochemical and Biophysical Research Communications, 125 - (2), 441 - 447 (1984); Bone, 8, 305 - 313 (1987)].

Under these circumstances, it has been proposed to apply certain cysteine protease inhibitors, which are expected to simultaneously suppress the decreases in calcium salts and collagen fibers, in order to treat bone resorption diseases (Japanese Patent Laid-Open No. 281427/1988 and No. 218610/1990). However studies aimed at achieving this have just been initiated so that no clinical cases where patients were cured by such an suppression have been reported. Thus no practical therapeutic agent of this type has been provided so far.

It is an object of the present invention to provide an agent for the preparation of a pharmaceutical composition useful for preventing or treating bone resorption diseases such as malignant hypercalcemia, bone Paget's disease or osteoporosis. More particularly, it aims at providing an agent which enables causative prevention and treatment of bone resorption diseases by suppressing the decrease in calcium salts in bones, the aforesaid agent being expected to substitute for conventional therapeutic compositions comprising supplying or maintaining blood calcium

It is another object of the present invention to provide a therapeutic composition for bone diseases which enables more effective prevention and treatment of bone resorption diseases by simultaneously suppressing the decreases in collagen fibers and calcium salts in bones.

A number of compounds were screened so as to find those capable of normalizing blood calcium concentration, by using rat models of hypercalcemia induced by PTHrp(1-34) and secondary hyperparathyroidism generated by a calcium-deficient diet. It was found that a class of compounds represented by the following general formula (I):

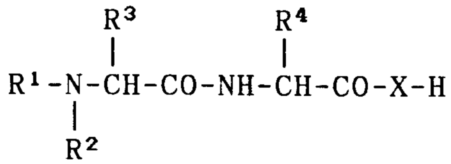

(I)

wherein $R^1$ represents a straight or branched acyl group having 2 to 10 carbon atoms, a branched cyclic or bridged cyclic alkyloxycarbonyl group having 4 to 15 carbon atoms, a substituted or unsubstituted benzyloxycarbonyl group, a 2,2,2-trichloroethyloxycarbonyl group, a 2-(trimethylsilyl)-ethyloxycarbonyl group, a p-toluenesulfonyl group, an o-nitrophenylsulfenyl group, a diphenylphosphonothioyl group, a triphenylmethyl group, a 2-benzoyl-1-methylvinyl group or a 4-phenylbutanoyl group;
$R^2$ represents a hydrogen atom or forms a phthaloyl group together with $R^1$;
$R^3$ represents an isobutyl group, an n-butyl group or an isopropyl group;
$R^4$ represents an n-butyl group or an isobutyl group;
X represents a direct bond, a methionine residue, a leucine residue or a norleucine residue; and
H at the C-terminal means that the carboxyl group is reduced in the form of the aldehyde;
which is capable of decreasing blood calcium concentration when administered orally or parenterally (for example, via intravenous injection), and thus can be used for the preparation of a pharmaceutical composition useful as a prophylactic and/or therapeutic agent for bone diseases.

The above-mentioned PTHrp(1-34) is an active fragment consisting of the 1 - 34 amino acid residues of PTHrp (parathyroid hormone-related protein) which is the factor responsible for inducing human malignant hypercalcemia. PTHrp(1-34) induces hypercalcemia when administered to rats, and promotes in vitro bone resorption as well [Journal of Clinical Investigation, 81, (2), 596 - 600 (1988); and Endocrinology, 123, 2841 - 2848 (1988)]. Thus this substance was used for obtaining an osteoporosis model system.

Compounds of the general formula (I) were administered to Wistar strain rats and PTHrp(1-34) was then given to the animals and the blood calcium concentration was measured. As a result, it was found that test groups showed a significantly reduced blood calcium concentration compared with the control group which received PTHrp(1-34) alone.

Secondary hyperparathyroidism is observed in some cases of bone resorption diseases. This is thought to be attributable to the acceleration of parathyroid hormone secretion which in turn takes place to maintain the blood calcium concentration within a normal range. In order to mimic such a hyperparathyroidism, rats were fed on a calcium-deficient diet so as to accelerate the secretion of parathyroid hormone to thereby induce secondary hyperparathyroidism.

By using this model system, the effects of compounds of the general formula (I) on secondary hyperparathyroidism were examined. As a result, it was found that the compounds significantly decreased blood calcium concentration which had been elevated by the calcium-deficient diet.

Accordingly, it has been proven that the compounds of the general formula (I) are effective on the above-mentioned two model systems of bone diseases, which indicates that they are useful as a prophylactic or therapeutic agent for bone diseases.

Furthermore, it is known that compounds of the general formula (I) in the form of a dipeptide potently inhibit cathepsin L (Japanese Patent Laid-Open No. 121257/1989 and No. 268145/1990). Also, compounds of the general formula (I) in the form of a tripeptide potently inhibit cysteine proteases and also inhibit cathepsins. On the other hand, it was reported that cathepsin L decomposed collagens type II, IX and XI, which constituted arthrodial cartilages, in a neutral pH range [FEBS Letters, 269 (1), 189 - 193 (1990)]. Thus the compounds of the general formula (I) are considered to be useful in the preparation of a pharmaceutical composition which is effective as a prophylactic or therapeutic agent for bone diseases, also from the view point that they are capable of potently inhibiting said decomposition. It is expected, therefore, that the pharmaceutical composition prepared according to the present invention will be further applicable for treatment of bone arthritis and rheumatic arthritis which are bone diseases accompanied by abnormal sthenia of cathepsin L.

Among compounds of the general formula (I), those in the form of a dipeptide are represented by the general formula (II) shown below. These compounds are disclosed in Japanese Patent Laid-Open No. 121257/1989 and No. 268145/1990. They can be prepared by organic synthesis disclosed in the laid open patent applications as cited above. Although these compounds are known to potently inhibit cathepsin L, it has not been known that they are effective on bone diseases.

Examples of preferred compounds of the general formula (II) as the active ingredient of the pharmaceutical compositions of the present invention include those where $R^4$ represents n-butyl group and $R^1$, $R^2$ and $R^3$ represent groups as listed in the following Table 1.

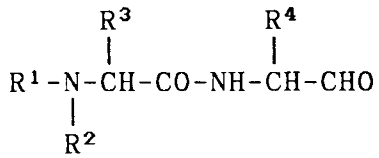

(II)

Table 1: Examples of compounds

| Compound No. | $R^1$ | $R^2$ | $R^3$ |
|---|---|---|---|
| 1* | benzyloxycarbonyl | H | isobutyl |
| 2* | 4-phenylbutanoyl | H | isobutyl |
| 3 | octanoyl | H | isobutyl |
| 4 | hexanoyl | H | isobutyl |
| 5 | isovaleryl | H | isobutyl |
| 6 | t-butoxycarbonyl | H | isobutyl |
| 7 | adamantyloxycarbonyl | H | isobutyl |
| 8 | p-chlorobenzyloxycarbonyl | H | isobutyl |
| 9 | p-methoxybenzyloxycarbonyl | H | isobutyl |
| 10 | o-chlorobenzyloxycarbonyl | H | isobutyl |
| 11 | 2,2,2-trichloroethyloxycarbonyl | H | isobutyl |
| 12 | 2-trimethylsilylethyloxycarbonyl | H | isobutyl |
| 13 | p-toluenesulfonyl | H | isobutyl |
| 14 | o-nitrophenylsulfenyl | H | isobutyl |
| 15 | diphenylphosphonothioyl | H | isobutyl |
| 16 | triphenylmethyl | H | isobutyl |
| 17 | 2-benzoyl-1-methylvinyl | H | isobutyl |
| 18 | phthaloyl | ($R^1$ and $R^2$ together) | isobutyl |
| 19 | benzyloxycarbonyl | H | n-butyl |

*Compounds 1 and 2 are disclosed in Japanese Patent Laid-Open No. 121257/1989, while the rest of the compounds are disclosed in Japanese Patent Laid-Open No. 268145/1990.

The active compound of the invention can be a tripeptide when X in the general formula (I) does not represent direct bond.

In one preferred group of tripeptides of the general formula (I), $R^1$ is acetyl group, $R^2$ is hydrogen, and $R^3$ and $R^4$ are isobutyl. Thus, they are expressed by the following general formula (IV):

Ac-Leu-Leu-X-H (IV)

wherein Ac represents an acetyl group;
Leu represents a leucine residue;
X represents a methionine residue, a leucine residue or a norleucine residue; and
H means that the C-terminal carboxyl group of the peptide is reduced in the form of the aldehyde.
Compounds of the general formula (VI) are disclosed as a cysteine protease inhibitor in Japanese Patent Laid-Open No. 103897/1986 by the present inventors, together with the method of chemical synthesis thereof.

In another preferred group of tripeptides of the general formula (I), R' is a benzyloxycarbonyl group, $R^2$ is hydrogen, and $R^3$ and $R^4$ are isobutyl. Thus, they are represented by the following general formula (V):

Z-Leu-Leu-X-H (V)

wherein Z represents a benzyloxycarbonyl group;
Leu represents a leucine residue;
X represents a methionine residue, a leucine residue or a norleucine residue; and
H means that the C-terminal carboxyl group of the peptide is reduced in the form of the aldehyde.
Compounds of the general formula (V) are disclosed as a neuron dendrite extending substances in Japanese Patent Laid-Open No. 268196/1990, together with the method of chemical synthesis thereof.

However none of these patent applications describe that the compounds disclosed therein are effective for bone diseases. Therefore, the usage of these compounds for the preparation of a pharmaceutical composition for the prophylaxis or therapy of bone diseases was found for the first time by the present inventors.

Further, if some tripeptide compounds of the present invention are not described in the two laid open patent applications as cited above, they can be synthesized in accordance with the methods described therein.

Examples of preferred tripeptide compounds are as follows:
N-Acetyl-leucyl-leucyl-leucinal (Compound 20),
N-Acetyl-leucyl-leucyl-methioninal (Compound 21),
N-Acetyl-leucyl-leucyl-norleucinal (Compound 22), and
N-Benzyloxycarbonyl-leucyl-leucyl-norleucinal (Compound 23).

The compound of the general formula (I) of the present invention may be included in a pharmaceutical composition also in the form of a derivative which is converted to the corresponding C-terminal aldehyde in vivo, such as the acetal derivative.

The active ingredient of the present invention may be administered in the form of various preparations produced in a conventional manner. Examples of oral preparations include capsules, tablets, granules, fine granules, syrups and dry syrups. Examples of parenteral preparations include injections, suppositories such as rectal and vaginal suppositories, nasal preparations such as sprays and percutaneous preparations such as ointments and percutaneous absorption tapes.

The bone diseases to be treated with the pharmaceutical compositions of the present invention are bone resorption diseases, for example, malignant hypercalcemia, bone Paget's disease and osteoporosis. Generally, the active ingredient may be orally or parenterally administered to adult patients suffering from these bone diseases in a dose of from 0.1 to 1,000 mg/kg body weight (preferably from 1 to 100 mg/kg body weight, still preferably from 5 to 50 mg/kg body weight) once to three times per day. However the dose may be appropriately controlled depending on the age and condition of each patient. The dose may be elevated so long as the efficacy of the active ingredient can be observed, taking the safety data as given

below into consideration.

The toxicity of the compounds of the general formula (I), which are low molecular weight peptide derivatives, is negligibly low. When 2 g/kg of respective Compound 1 (N-benzyloxycarbonyl-leucyl-norleucinal) and Compound 22 (N-acetyl-leucyl-leucyl-norleucinal was intraperitoneally given to rats in order to examine the acute toxicity, neither any change in the general conditions of the animals nor any mortal case was observed.

## Examples

To further illustrate the present invention in greater detail, the following non-limiting examples are given.

### Example 1

Effects of the prophylactic or therapeutic agent of the present invention on rat hypercalcemia induced by PTHrp(1-34) (oral administration):

To male Wistar rats aged 5 weeks (70 - 90 g, 5 animals in each group), 40 mg/kg body weight of the compounds listed in Table 2 and 40 and 80 mg/kg body weight of the compounds listed in Table 3 were orally administered. After 4 hours, 5 nmole/kg of PTHrp(1-34) was intravenously injected into the animals.

One hour after the administration of PTHrp(1-34), a blood sample was collected from each rat and the blood calcium concentration was determined by the octocresolphthalein complexone method.

A comparative compound, E-64 from Japanese Patent Laid-Open No. 284127/1988 was orally administered at the same doses as those for the test compounds and the effect was evaluated in the same manner.

The results which are given in Table 2 show that the compounds of the present invention significantly suppressed the increase in blood calcium concentration.

These results strongly suggest that the compounds of the present invention are effective in preventing and suppressing malignant humoral hypercalcemia. Thus it has been established that the compounds of the present invention are useful as a prophylactic or therapeutic agent for bone diseases.

Table 2: Effects on hypercalcemia (oral administration)

| Group | PTHrp-treatment | Drug | Dose (mg/kg) | Ca conc. ± standard error (mg/dl) |
|---|---|---|---|---|
| 1 | none | none | 0 | 10.41 ± 0.07 |
| 2 | yes | none | 0 | 11.19 ± 0.04 |
| 3 | yes | E-64 | 40 | 10.50 ± 0.23 |
| 4 | yes | cpd. 1 | 40 | 8.88 ± 0.13**## |
| 5 | yes | cpd. 2 | 40 | 10.56 ± 0.13** |
| 6 | yes | cpd. 3 | 40 | 10.52 ± 0.16** |
| 7 | yes | cpd. 5 | 40 | 9.76 ± 0.18**# |
| 8 | yes | cpd. 6 | 40 | 10.48 ± 0.13** |
| 9 | yes | cpd. 7 | 40 | 10.48 ± 0.29* |
| 10 | yes | cpd. 8 | 40 | 9.39 ± 0.15**## |
| 11 | yes | cpd. 9 | 40 | 9.24 ± 0.18**## |
| 12 | yes | cpd.10 | 40 | 10.46 ± 0.11** |
| 13 | yes | cpd.11 | 40 | 9.15 ± 0.05**## |
| 14 | yes | cpd.12 | 40 | 10.62 ± 0.23 |
| 15 | yes | cpd.13 | 40 | 9.77 ± 0.18**# |
| 16 | yes | cpd.15 | 40 | 9.76 ± 0.14**# |
| 17 | yes | cpd.16 | 40 | 10.59 ± 0.12* |
| 18 | yes | cpd.17 | 40 | 11.16 ± 0.12 |
| 19 | yes | cpd.18 | 40 | 10.72 ± 0.07** |
| 20 | yes | cpd.19 | 40 | 10.61 ± 0.07** |

E-64: L-trans-epoxysuccinyl-L-leucineamide-(e-guanide)-butane.
Average blood calcium concentration ± standard error (n = 5)
* : P < 0.05 vs. blood calcium concentration of Group 2.
**: P < 0.01 vs. blood calcium concentration of Group 2.
# : P < 0.05 vs. blood calcium concentration of Group 3.
##: P < 0.01 vs. blood calcium concentration of Group 3.

Table 3

| Effects on hypercalcemia (oral administration) | | | | |
|---|---|---|---|---|
| Group | PTHrp-treatment | Drug | Dose (mg/kg) | Ca conc. ± standard error (mg/dl) |
| 1 | none | none | 0 | 9.76 ± 0.06 |
| 2 | yes | none | 0 | 10.33 ± 0.13 |
| 3 | yes | E-64 | 40 | 9.69 ± 0.21* |
| 4 | yes | E-64 | 80 | 9.61 ± 0.12** |
| 5 | yes | cpd. 21 | 40 | 9.78 ± 0.08** |
| 6 | yes | cpd. 21 | 80 | 9.61 ± 0.13** |
| 7 | yes | cpd. 22 | 40 | 9.49 ± 0.20** |
| 8 | yes | cpd. 22 | 80 | 9.52 ± 0.11** |
| 9 | yes | cpd. 23 | 40 | 9.53 ± 0.08** |
| 10 | yes | cpd. 23 | 80 | 9.28 ± 0.66**# |

E-64: L-trans-epoxysuccinyl-L-leucineamide-(e-guanide)-butane.
Average blood calcium concentration ± standard error (n = 5)

* : P < 0.05 vs. blood calcium concentration of Group 2.
**: P < 0.01 vs. blood calcium concentration of Group 2.
# : P < 0.05 vs. blood calcium concentration of Group 4.

Example 2

Effects of the prophylactic or therapeutic agent of the present invention on rat hypercalcemia induced by PTHrp(1-34) (intraperitoneal administration):

To male Wistar rats aged 5 weeks (70 - 90 g, 5 animals in each group), 10 mg/kg body weight of Compound 1 was intraperitoneally administered 0.5, 1, 2 and 4 hours before the administration of PTHrp(1-34). PTHrp(1-34) was intravenously injected into the animals at the level of 5 nmole/kg. One hour after the administration of PTHrp(1-34), a blood sample was collected from each animal and the blood calcium concentration was determined by the same method as the one employed in Example 1.

Table 4 summarizes the results. The results show that Compound 1 continuously effected significant suppression of the increase in the blood calcium concentration. These results prove that the compound of the present invention is also effective in preventing and suppressing malignant humoral hypercalcemia, when administered parenterally and that this effect is sustained.

Table 4

| Effects on hypercalcemia (intraperitoneal administration) | | | | |
|---|---|---|---|---|
| Group | Drug-administration time (before PTH-treatment | Drug | Dose (mg/kg) | Ca conc. ± standard error (mg/dl) |
| 1 | 0.5 hr | none | 0 | 12.08 ± 0.11 |
| 2 | 0.5 hr | cpd. 1 | 10 | 11.74 ± 0.07* |
| 3 | 1 hr | none | 0 | 11.53 ± 0.08 |
| 4 | 1 hr | cpd. 1 | 10 | 11.28 ± 0.07* |
| 5 | 2 hr | none | 0 | 11.99 ± 0.04 |
| 6 | 2 hr | cpd. 1 | 10 | 11.56 ± 0.09** |
| 7 | 4 hr | none | 0 | 12.46 ± 0.07 |
| 8 | 4 hr | cpd. 1 | 10 | 11.91 ± 0.09** |
| Average blood calcium concentration ± standard error (n = 5) | | | | |

* : P < 0.05 vs. blood calcium concentration of control group at each point.
**: P < 0.01 vs. blood calcium concentration of control group at each point.

Example 3

Effects of secondary hyperparathyroidism:

In a calcium-deficient diet model system of rats, parathyroid hormone is released in order to maintain the blood calcium concentration within a normal range. In other words, the parathyroid hormone induces bone resorption so that the blood calcium concentration is maintained within a normal range. A great portion of the calcium in the blood in these animals has been found to be dissolved from bones.

Taking advantage of the above-mentioned model system, male Wistar rats aged 5 weeks (70 - 90 g, 5 animals in each group) were fed on calcium-deficient diet for 5 days. After fasting overnight, 40 mg/kg body weight of the compounds listed in Table 1 were orally administered to the rats. 5 hours after the administration of the drugs, the blood calcium concentration of each animal was determined by the same method as the one employed in Example 1.

Table 5 summarizes the results. The results show that Compounds 2, 6, 8, 9, 11 and 15 significantly reduced the blood calcium concentration. Thus it was confirmed that the therapeutic agent of the present invention is also effective in the prevention and treatment of bone diseases accompanied by secondary hyperparathyroidism.

Table 5: Effects on secondary hyperparathyroidism

| Group | Drug | Dose (mg/kg) | Ca conc. ± standard error (mg/dl) |
|---|---|---|---|
| 1 | none | 0 | 11.06 ± 0.19 |
| 2 | cpd. 1 | 40 | 11.30 ± 0.26 |
| 3 | cpd. 2 | 40 | 10.38 ± 0.17* |
| 4 | cpd. 3 | 40 | 10.94 ± 0.05 |
| 5 | cpd. 5 | 40 | 10.70 ± 0.13 |
| 6 | cpd. 6 | 40 | 10.22 ± 0.14** |
| 7 | cpd. 7 | 40 | 10.54 ± 0.16 |
| 8 | cpd. 8 | 40 | 10.45 ± 0.14* |
| 9 | cpd. 9 | 40 | 10.43 ± 0.09* |
| 10 | cpd.10 | 40 | 10.38 ± 0.16* |
| 11 | cpd.11 | 40 | 10.39 ± 0.08** |
| 12 | cpd.12 | 40 | 9.90 ± 0.63 |
| 13 | cpd.13 | 40 | 10.74 ± 0.04 |
| 14 | cpd.15 | 40 | 9.99 ± 0.12** |
| 15 | cpd.16 | 40 | 10.61 ± 0.19 |
| 16 | cpd.17 | 40 | 10.73 ± 0.12 |
| 17 | cpd.18 | 40 | 10.60 ± 0.09 |
| 18 | cpd.19 | 40 | 10.91 ± 0.20 |

Average blood calcium concentration ± standard error (n = 5)
* : $P < 0.05$ vs. blood calcium concentration of Group 2.
**: $P < 0.01$ vs. blood calcium concentration of Group 2.

Formulation Example 1

Production of capsules:

| | | |
|---|---|---|
| Compound 1 | 100 | (parts by weight) |
| potato starch | 148 | " |
| magnesium stearate | 2 | " |

The above ingredients were thoroughly mixed in an agitator and put in No. 1 hard gelatin capsules in 250 mg portions. Thus capsules each containing 100 mg of Compound 1 were obtained.

Formulation Example 2

Production of rectal suppositories:

Witepsol H-15 (Dynamic Nobel) was melted by heat and Compound 1 was added thereto so as to give a concentration of 12.5 mg/ml. After homogeneously mixing, 2 ml portions of the mixture were poured into a rectal suppository mold and cooled. Thus rectal suppositories each containing 25 mg of Compound 1 were obtained.

Formulation Example 3

Production of tablets:

| | |
|---|---|
| Compound 22<br>microcrystalline cellulose<br>magnesium stearate | 100 g<br>22.5 g<br>2.5 g |
| Total | 125.0 g |

The above ingredients were mixed together and punched into tablets by a tablet machine. Thus tablets (diameter: 9 mm, weight: 250 mg) each containing 200 mg of Compound 22 were obtained.

Formulation Example 4

Production of rectal suppositories:

Witepsol H-15 was melted by heat and Compound 22 was added thereto so as to give a concentration of 12.5 mg/ml. After homogeneously mixing, 2 ml portions of the mixture were poured into a rectal suppository mold and cooled. Thus rectal suppositories each containing 25 mg of Compound 22 were obtained.

The active ingredients of the present invention significantly reduce blood calcium concentration in rat hypercalcemia which is a model system of bone resorption diseases induced with PTHrp(1-34). Accordingly, these compounds are useful as a prophylactic or therapeutic agent for bone diseases.

## Claims

**1.** Use of a compound represented by the following general formula (I):

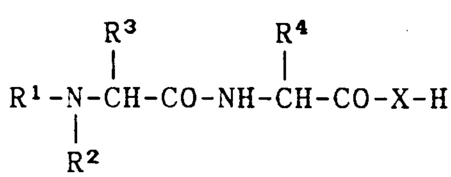

(I)

wherein $R^1$ represents a straight or branched acyl group having 2 to 10 carbon atoms, a branched cyclic or bridged cyclic alkyloxycarbonyl group having 4 to 15 carbon atoms, a substituted or unsubstituted benzyloxycarbonyl group, a 2,2,2-trichloroethyloxycarbonyl group, a 2-(trimethylsilyl)-ethyloxycarbonyl group, a p-toluenesulfonyl group, an o-nitrophenylsulfenyl group, a diphenylphosphonothioyl group, a triphenylmethyl group, a 2-benzoyl-1-methylvinyl group or a 4-phenylbutanoyl group;
$R^2$ represents a hydrogen atom or forms a phthaloyl group together with $R^1$;
$R^3$ represents an isobutyl group, an n-butyl group or an isopropyl group;
$R^4$ represents an n-butyl group or an isobutyl group;
X represents a direct bond, a methionine residue, a leucine residue or a norleucine residue; and
H at the C-terminal means that the carboxyl group is reduced in the form of the aldehyde;
for the preparation of a pharmaceutical composition useful in the prophylaxis or therapy of bone diseases.

2. Use as claimed in Claim 1, comprising a compound wherein X is a direct bond represented by the following general formula (II):

```
        R3          R4
        |           |
R1-N-CH-CO-NH-CH-CHO                    (II)
   |
   R2
```

wherein $R^1$ represents a straight or branched acyl group having 2 to 10 carbon atoms, a branched cyclic or bridged cyclic alkyloxycarbonyl group having 4 to 15 carbon atoms, a substituted or unsubstituted benzyloxycarbonyl group, a 2,2,2-trichloroethyloxycarbonyl group, a 2-(trimethylsilyl)-ethyloxycarbonyl group, a p-toluenesulfonyl group, an o-nitrophenylsulfenyl group, a diphenylphosphonothioyl group, a triphenylmethyl group, a 2-benzoyl-1-methylvinyl group or a 4-phenylbutanoyl group;
$R^2$ represents a hydrogen atom or forms a phthaloyl group together with $R^1$;
$R^3$ represents an isobutyl group, an n-butyl group or an isopropyl group; and
$R^4$ represents an n-butyl group;
as an active ingredient.

3. Use as claimed in Claim 1, comprising a compound represented by the following general formula (III):

R-Leu-Leu-X-H (III)

wherein R represents an acetyl group or benzyloxycarbonyl group;
Leu represents a leucine residue;
X represents a methionine residue, a leucine residue or a norleucine residue; and
H means that the C-terminal carboxyl group of the peptide is reduced in the form of the aldehyde.

4. Use as claimed in any of Claims 1 to 3, wherein said bone disease is selected from the group consisting of malignant hypercalcemia, bone Paget's disease or osteoporosis.

**Claims for the following Contracting States : ES, GR**

1. A method for the preparation of a pharmaceutical composition useful for the prophylaxis or therapy of bone diseases, which comprises combining a compound represented by the following general formula (I):

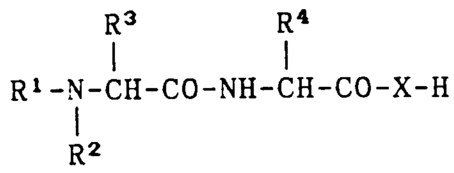

(I)

wherein $R^1$ represents a straight or branched acyl group having 2 to 10 carbon atoms, a branched cyclic or bridged cyclic alkyloxycarbonyl group having 4 to 15 carbon atoms, a substituted or unsubstituted benzyloxycarbonyl group, a 2,2,2-trichloroethyloxycarbonyl group, a 2-(trimethylsilyl)-ethyloxycarbonyl group, a p-toluenesulfonyl group, an o-nitrophenylsulfenyl group, a diphenylphosphonothioyl group, a triphenylmethyl group, a 2-benzoyl-1-methylvinyl group or a 4-phenylbutanoyl group;
$R^2$ represents a hydrogen atom or forms a phthaloyl group together with $R^1$;
$R^3$ represents an isobutyl group, an n-butyl group or an isopropyl group;
$R^4$ represents an n-butyl group or an isobutyl group;
X represents a direct bond, a methionine residue, a leucine residue or a norleucine residue; and
H at the C-terminal means that the carboxyl group is reduced in the form of the aldehyde;
with a pharmaceutically acceptable carrier.

2. A method as claimed in Claim 1, comprising a compound wherein X is a direct bond represented by the following general formula (II):

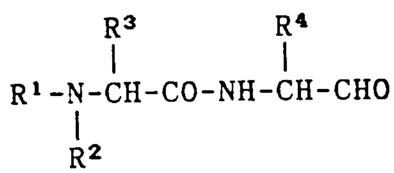

(II)

wherein $R^1$ represents a straight or branched acyl group having 2 to 10 carbon atoms, a branched cyclic or bridged cyclic alkyloxycarbonyl group having 4 to 15 carbon atoms, a substituted or unsubstituted benzyloxycarbonyl group, a 2,2,2-trichloroethyloxycarbonyl group, a 2-(trimethylsilyl)-ethyloxycarbonyl group, a p-toluenesulfonyl group, an o-nitrophenylsulfenyl group, a diphenylphosphonothioyl group, a triphenylmethyl group, a 2-benzoyl-1-methylvinyl group or a 4-phenylbutanoyl group;
$R^2$ represents a hydrogen atom or forms a phthaloyl group together with $R^1$;
$R^3$ represents an isobutyl group, an n-butyl group or an isopropyl group; and
$R^4$ represents an n-butyl group.

3. A method as claimed in Claim 1, comprising a compound represented by the following general formula (III):

R-Leu-Leu-X-H (III)

wherein R represents an acetyl group or a benzyloxycarbonyl group;
Leu represents a leucine residue;
X represents a methionine residue, a leucine residue or a norleucine residue; and
H means that the C-terminal carboxyl group of the peptide is reduced in the form of the aldehyde.

4. A method as claimed in any of Claims 1 to 3, wherein said bone disease is selected from the group consisting of malignant hypercalcemia, bone Paget's disease or osteoporosis.